# EUROPEAN PATENT SPECIFICATION

(11) **EP 3 142 033 B1**
(45) Date of publication and mention of the grant of the patent: **20.07.2022**
(21) Application number: 16188087.7
(22) Date of filing: 09.09.2016
(51) Int. Cl.: G16H 50/20, G16H 50/50

(54) **PHYSIOLOGY-DRIVEN DECISION SUPPORT FOR THERAPY PLANNING**
PHYSIOLOGIEGESTEUERTE ENTSCHEIDUNGSUNTERSTÜTZUNG ZUR THERAPIEPLANUNG
SUPPORT DE DÉCISION GUIDÉ PAR LA PHYSIOLOGIE POUR UNE PLANIFICATION DE THÉRAPIE

(30) Priority: 11.09.2015 US 201514851552
(43) Date of publication of application: 15.03.2017
(73) Proprietor: Siemens Healthcare GmbH, 91052 Erlangen (DE)
(72) Inventor: Georgescu, Bogdan, Plainsboro, NJ New Jersey 08536 (US); Mansi, Tommaso, Plainsboro, NJ New Jersey 08536 (US); Neumann, Dominik, 91052 Erlangen (DE); Ecabert, Olivier, 91320 Ebermannstadt (DE); Mihalef, Viorel, North Brunswick, NJ New Jersey 08902 (US); Passerini, Tiziano, Plainsboro, NJ New Jersey 08536 (US); Pauly, Olivier, 81241 München (DE)
(74) Representative: Bals & Vogel Patentanwälte PartGmbB

(56) References cited:
- US-A1- 2005 131 663
- US-A1- 2010 280 352
- US-A1- 2015 242 589
- CASEY C. BENNETT ET AL: "Artificial intelligence framework for simulating clinical decision-making: A Markov decision process approach", ARTIFICIAL INTELLIGENCE IN MEDICINE, vol. 57, no. 1, 1 January 2013 (2013-01-01), pages 9-19, XP055256441, NL ISSN: 0933-3657, DOI: 10.1016/j.artmed.2012.12.003

## Description

### Background

The present embodiments relate to decision support for therapy planning. In therapy planning, many decisions need to be made by the clinicians, guided through guidelines but very often driven by experience. For example, in stenting procedures, there are typically many different stents from which to choose. The different stents have different parameters, such as diameter, length, porosity, metal coverage area, pore shape, and/or material mechanical properties. There are likewise different options for positioning the stent inside the vessel. Due to the limited available information and complexity of the anatomical structures, of the disease and of the procedure itself, the outcome of a specific therapy may not always be anticipated by the clinician. Having reliable predication of outcome may help in planning and performing the optimal therapy for the patient under consideration.

Recently, computational modeling of physiological systems has been developed. Because such methods are predictive, through simulation, the modeling may be used to test therapy *in-silico.* However, with the multiplication of models and clinical data available for a patient, the options make computational assistance overwhelming. CASEY C. BENNETT ET AL: "Artificial intelligence framework for simulating clinical decision-making: A Markov decision process approach" discloses a method for clinical decision making using Al. This document outlines a general method for developing an AI framework (in particular through Markov models) disclosing a clinical decision method based on an outcome data bank and represents the closest prior art. This document does not discose the application of an autoencoder and the determination of nearest neighbour patients for decision making in patients needing pulmonary artery stenting, cardiac resynchronisation planning, ablation therapy or valve replacement.

### Summary

Systems, methods, and computer readable media are provided for decision support for therapy. Using computational models for the patient physiology and the various therapy options, a decision support system presents a range of predicted outcomes to assist in planning the therapy. The models are used in various experiments for the many therapy options to determine an optimal approach. The present invention discloses a computer implemented method as defined by claim 1.

In a first aspect, a method is provided for decision support for therapy. Organ data representing an organ of a first patient is segmented from scan data from a medical scanner. The scan data represents a volume of the first patient. A processor simulates a plurality of different therapies with a physiological model personalized to the organ based on the segmented data. The different therapies are for a therapy device with different parameters and/or for different therapy devices. The processor estimates uncertainties in the simulated outcomes of the different therapies. The simulated outcomes of the simulating of the different therapies and the estimated uncertainties are presented on a display.

In a second aspect, a method is provided for decision support for therapy. Patient information is input from different sources to a first deep auto-encoder. The patient information is specific to a first patient and a type of therapy device. Similar patients to the first patient are selected with an output of the first deep auto-encoder. The similar patients have been treated with the type of therapy device. A range of outcomes is inferred from a range of therapy devices of the type of therapy device from data for the similar patients. The range of outcomes and the range of therapy devices for the first patient are displayed.

In a third aspect, a method is provided for decision support for therapy. Patient information is input from different sources to a first deep auto-encoder. The patient information is specific to a first patient and a type of therapy device. First similar patients to the first patient are selected with an output of the first deep auto-encoder. The similar patients have been treated with the type of therapy device. A first range of first outcomes is inferred from a range of therapy devices of the type of therapy device from data for the first similar patients. At least one of the therapy devices is elected based on the outcome. Treatment by the selected at least one of the therapy devices is simulated using a physiological model personalized to the first patient and a model of the type of therapy device specific to the at least one of the therapy devices. Hemodynamic factors resulting from the simulation of the treatment are calculated. The hemodynamic factors and at least some of the patient information are input to a second deep auto-encoder. Second similar patients to the first patient are selected with an output of the second deep auto-encoder. At least one second outcome is inferred from the at least one of the therapy devices from data for the second similar patients. The at least one second outcome and the at least one therapy device for the first patient are displayed.

Any one or more of the aspects described above may be used alone or in combination. These and other aspects, features, and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### Brief Description of the Drawings

The components and the figures are not necessarily to scale, emphasis instead being placed upon illustrating the principles of the embodiments. Moreover, in the figures, like reference numerals designate corresponding parts throughout the different views.
Figure 1 shows a flow chart of one embodiment of a method for decision support in therapy;
Figure 2 illustrates another embodiment of a method for decision support in therapy;
Figure 3 shows one embodiment of a method for decision support where outcome inference is through nearest neighbor retrieval;
Figure 4 illustrates an example deep auto-encoder;
Figure 5 shows another embodiment of a method for decision support where outcome inference is through nearest neighbor and modeling; and
Figure 6 is a block diagram of one embodiment of a system for decision support in therapy.

### Detailed Description of Embodiments

A physiology-driven decision support system may assist in therapy planning for specific patients. The decision support system is an objective, automated system that guides virtual experiments to be carried out and summarizes the results for therapy decision support. Simulated information is integrated with patient clinical data and history in an intuitive fashion. The clinical decision support system is driven by models of physiological systems and advanced machine learning techniques to help the clinician select the therapy from among a set of available options for a given patient and procedure.

The decision support system launches therapy simulations according to 1) user-defined scenario, 2) clinical board scenario or standard operating procedures (SOP) from a hospital or a specialty community, or 3) clinical guidelines. The therapies may be simulated on local hardware, such as a workstation, or in the cloud. The results of the simulation are presented on a summary page, enabling the user to visualize the outputs of the simulations. In the case of virtual stenting, for instance, the visualized information may include screenshots or videos of simulated blood flow after stent deployment, numerical or visual indicators of vessel wall deformation, predicted stent load, computed pressure change, or other hemodynamics information. The decision support system serves as a review tool for the clinician and may help the clinician in selecting the best treatment option based on the coherent data provided by the decision support system. Suggestions may be presented (i.e. the top two therapy options according to user-defined criterion). Therapy options yielding similar outcomes, such as according to user-defined criterion, are gathered automatically in one result set to help the review process.

In one embodiment, patient clinical data and images are loaded. The organ of interest is segmented. A virtual experiment that involves computing a set of therapy simulations given physiological models, therapy models, and therapy parameters is designed. The physiological models are personalized from patient data. The simulation uncertainty is estimated. Simulation results are gathered and ranked according to any metric of interest. Results with similar outcome may be clustered. The results are presented with their confidence if the uncertainty could be calculated. The user may then be asked to select the best option. Machine learning may be used to learn the virtual experiment design process and expected outcomes from the user in a reinforcement fashion and/or from a database of clinical case reports and guidelines.

In the discussion below, the operation of the decision support system is described in a use case of pulmonary artery (PA) stenting, cardiac resynchronization planning, ablation therapy (cardiac arrhythmias or tumors), or valve replacement.

The pulmonary artery sends low-oxygen blood from the right ventricle of the heart to the lungs, where the blood is enriched with oxygen to fulfill the needs of the entire body and organs. However, due to various factors (e.g. congenital), a narrowing may occur which limits the amount of blood passing through the PA. This narrowing is called PA stenosis. If severe and untreated, PA stenosis may lead to significantly increased pressure in the right ventricle, which in turn may cause irreversible damage to the myocardium. A common way to treat PA stenosis is through transcatheter balloon angioplasty and/or stenting. The aim of the intervention is to dilate and stabilize the narrowed segment. The outcome, however, depends on the size, type, and deployment parameters of the selected stent and may vary from patient to patient. Moreover, this therapy has an estimated 22% risk of associated procedure-related adverse events, ranging from vascular or cardiac trauma (e.g. tear), to arrhythmias, to balloon rupture, or to stent embolization. In 10% of the cases, adverse events are severe or fatal. Therefore, personalized treatment is crucial and treatment planning through advanced computational model simulations desirable as the decision support system may lead to more refined therapy decision not possible with manual efforts of a physician.

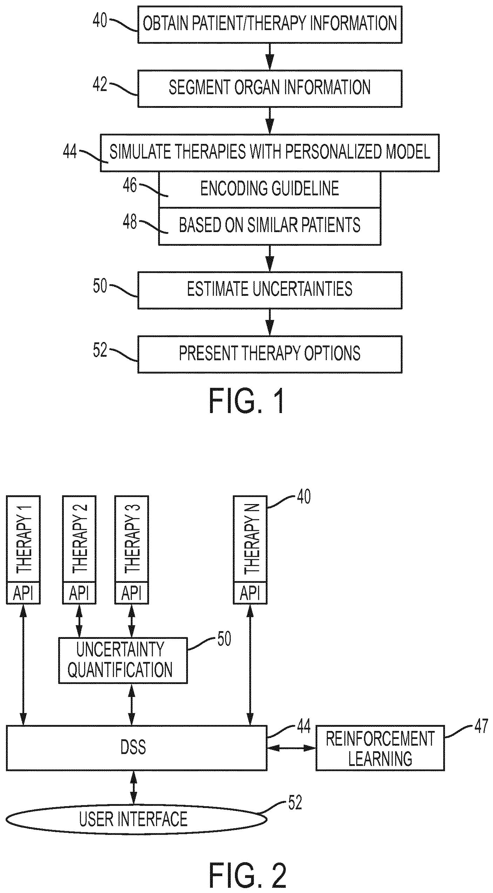

Figure 1 shows a flow chart for one embodiment of a method for decision support therapy. Personalized modeling is used to simulate therapies. The simulation may be performed with the personalized model and therapy models. The interaction of the models provides an indication of effectiveness of therapies for the patient. In other approaches, the simulation uses modeling through machine-learnt selection of patients modeled as being similar to a given patient. The different therapies and resulting outcomes from those similar patients are used to indicate effectiveness of therapies for the patient. The outcomes of the simulated therapies are presented to the user to assist in deciding on therapy for a given patient.

Figure 2 shows another embodiment of the method for decision support for therapy. The input to the decision support system is tools for modeling the different available therapies. The decision support system compiles the predicted outcome from all therapies, including uncertainties if available. The outcomes are presented to the clinician in a visual user interface. Reinforcement learning or other learning techniques may be applied to automatically compute the optimal therapy according to pre-defined or learned objective criteria.

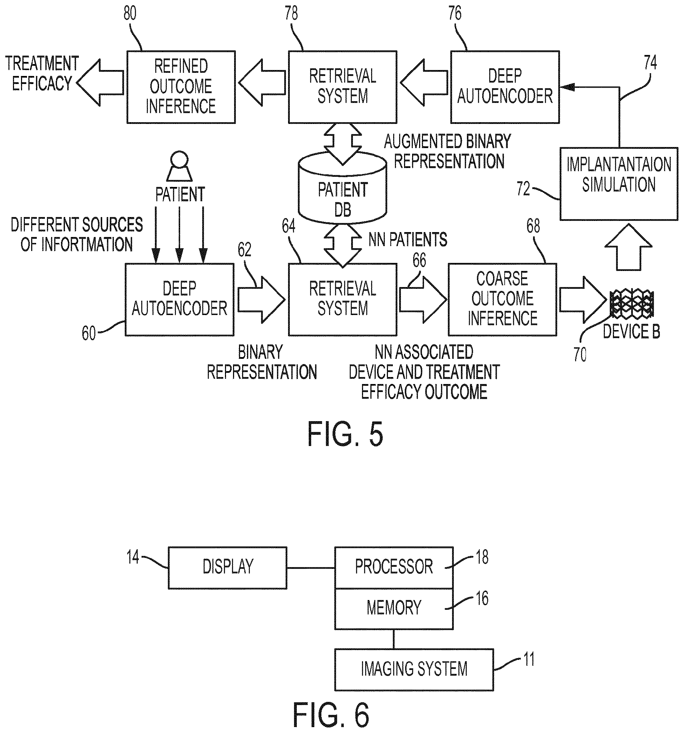

The methods are implemented on the system of Figure 6 or a different system. A computer, workstation, server, or other processor receives or obtains data for a patient and uses that information to simulate application of different therapies on the patient. A database stores models, current patient information, past patient information, or other data accessed and used by the processor.

The methods are provided in the orders shown, but other orders may be provided. Additional, different or fewer acts may be provided. For example, acts 40 and/or 50 are not provided. As another example, act 52 is not provided. In yet other examples, acts for scanning a patient or user interaction are provided.

In act 40, a processor obtains information to be used for simulation. The information includes patient and/or therapy information. The information is obtained from a database, such as a computerized patient record or other memory. Alternatively, a user inputs the information with a user interface.

The patient information may include hemodynamic measures, clinical data, scan data, or combinations thereof. Additional, different, or fewer types of patient information may be provided.

Hemodynamic measures are extracted from a personalized model and/or from scan data. Hemodynamic measures include velocity, variance, volume flow, pressure, change in flow, change in pressure, differential pressure, and/or other measures of flow.

The patient information includes scan data. A medical scanner, such as a magnetic resonance imaging (MRI), computed tomography (CT), or ultrasound system, scans a patient. The scan data represents a two or three-dimensional region of the patient, such as scanning a vessel of the patient. The scan may be repeated to represent the region over time.

Other patient information includes clinical data. Any clinical data may be obtained, such as test results. Patient medical and/or family history may be included. Any combination of various types of patient information may be obtained.

Models are also obtained. The therapy information includes models of the therapies. One model that alters as a function of different parameters may be used to create models for different therapies. One or more models representing a part of the patient, such as vessel model, may be obtained. Each model may provide different information or may provide the same information but in a different way. The vessel model may be fit to the patient information, personalizing the model to the patient. The fit may be spatial or structural, such as sizing a mesh to the scan data for the patient. The fit may alternatively or additionally be by optimizing values of parameters, such as determining a pressure, flow rate, tissue elasticity, tissue modulus, flow rate, and/or other hemodynamic parameter that causes the model to best represent or fit the dynamic behavior of the anatomy over time.

For simulation or personalizing the physiological model, organ information is segmented from the scan data in act 42. The scan data representing the organ of interest (e.g., vessel) is identified. Voxels representing the volume of the patient that correspond to the organ are determined. Other scan data is ignored or masked out.

Where the scan data represents the patient over time, such as over one or more heart cycles, the segmentation may be performed for each of the times. Alternatively or additionally, the organ as segmented at one time may be tracked to other times in order to segment for the other times.

In one embodiment, in order to obtain the patient-specific vessel geometry, the vessel is segmented from medical images or scan data, like MRI, CT, interventional CT using a C-arm, or ultrasound scan data. Data-driven machine-learning based segmentation tools may be employed to facilitate and automate the segmentation process and thus make the results reproducible. Manual or programmed (e.g., intensity threshold or window/level based segmentation) may be used. If dynamic images are available, the vessel boundaries are tracked in each frame or time to get a time-resolved representation of the vessel dynamics.

In act 44, a processor simulates a plurality of different therapies. The therapies may be for the same type of device, such as a stent. By varying properties of the device, different therapies are provided. The size, shape, material characteristic, placement or position, stiffness, and/or other properties may be varied to provide different therapies. The different therapies may be for different types of devices, such as using a stent or using a balloon catheter without stenting. The different therapies may use the same device, but in different ways or through different workflows, providing different therapies.

Other therapies may be considered, like cardiac resynchronization therapy or ablation therapies. The different therapies may use one or more different types of devices and/or processes, providing different therapies.

The simulation of different therapies provides decision support. The decision support system is a case reasoning system based on rules, guidelines, retrieval of similar patients or decision-making learning techniques to support the selection of a therapy for optimal treatment. The simulation may be a simulation of interaction through use of models and/or a simulation by estimating outcome from similar patients' previous use of different therapies. Other types of simulation may be used. Combinations of types of simulation may be used.

The data of a current patient represents, in part, the patient's physiology, so is used in the simulation to personalize the physiological model. The simulations provide a virtual experiment of different therapies for a specific patient in an effort to identify the therapy or therapies that are more effected and/or have lesser side effects. In one embodiment, the decision support system is able to: 1) design a virtual experiment involving multiple simulations with multiple models and parameters, 2) launch the designed experiments, 3) aggregate and present the results, and 4) refine from its interaction with the user the intelligent components that enable 1).

The processor simulates the different therapies with a physiological model personalized to the organ based on the segmented data. The physiological model as personalized may provide values used for simulation (e.g., calculate hemodynamic factors used to find similar patients). Alternatively or additionally, the physiological model as personalized is used in a processor run simulation in interaction with a therapy model (e.g., stent model) to determine the physical, biological, electrical, or other interaction between the personalized model and the therapy model. In other embodiments, the simulation uses the physiology of the patient by finding similar patients.

The physiological model is personalized to a segmented organ of the patient and may be personalized, if available, with other data. The other data may include information from the image (e.g., motion and/or advanced physiological information such as tissue properties (stiffness) extracted from motion or measured by scanning). Other data may be non-imaging data, such as 12-lead ECG (electrocardiogram) and/or pressure in the ventricles and/or vessels from either catheterization or other devices to measure blood pressure (cuff).

More than one physiological model may be used for the simulation. Different models may provide different information or different values for the same parameters. The decision support system may help reduce complication by using the different models as different options (e.g., as different experiments). Alternatively, the different models are used to provide values that are averaged or are used together to provide information as inputs to the decision support system or outputs from simulation.

The physiological model provides biomechanical parameters. The biomechanical parameters may be tissue properties, such as elasticity or modulus. Shape or anatomical parameters may be provided, such as curvature, diameter, area, volume, size, or orientation. Hemodynamic parameters may be provided, such as pressure, differential pressure, flow, volume flow, change in flow or pressure, or other fluid dynamic information. Other types of parameters may be provided by the physiological model.

The physiological model provides the parameters using inverse modeling, computational fluid dynamics, or other physics modeling. In one embodiment, biomechanical parameters of a vessel are estimated in creating the personalized model. The moving boundaries obtained by segmenting over time or tracking are used to estimate the tissue properties of the vessel through inverse modeling techniques such as inverse optimization. In inverse optimization, the goal is to iteratively minimize the error between the output of a simulation of a computational model and corresponding clinical measurements by tuning the model parameters (e.g. vessel stiffness). For example, blood pressure data near the stenosis in the vessel is measured directly in the vessel using catheterization. The model is altered until providing the correct blood pressure. Alternatively, if such direct data is not available but instead the in-flow and out-flow (e.g., from MRI or ultrasound) data is available, computational fluid dynamics (CFD) simulations may be employed to compute the pressure gradient and surrogate pressure.

In an example embodiment, interaction between the physiological vessel model and the stent therapy model is solved computationally. The therapy models are provided as application program interfaces. Before starting the virtual stent deployment, the stent with specific properties (e.g., diameter, length, porosity, metal coverage area, pore shape, and/or material mechanical properties) is selected from a library of available devices (e.g., library of therapy device models). To model realistic physical properties of the selected device while still enabling fast computations, a mass-spring model may be used. Alternatively, stent model approximations based on deformable surfaces with regionally varying mechanical properties may be employed, depending on the required accuracy of the simulation and availability of virtual stent models.

Once the anatomical model is segmented or personalized to the segmented organ, the vessel tissue properties personalized, and the device selected, the stent is virtually deployed in a computer simulation. The entire deployment process, starting from stent crimping (packaging), followed by fitting into a microcatheter, then the maneuvering and delivery of the stent-microcatheter system, and finally stent release from the microcatheter, may be modeled. A reduced sequence of this whole procedure, which would include at least the final stent deployment, may instead be used.

The interaction is modeled based on physics, so may be solved computationally. In real stent deployment, a force is externally generated (e.g., typically using a balloon) to drive the deployment of the device. Other types of forces, like shape-memory, may be implemented by using virtual springs that are attached to the non-deployed stent and would deform the stent toward its original, deployed position. The typical geometry of the stent lends itself to being modeled as a network of interwoven fibers. An effective way of describing such a structure is based on reduced order (e.g., one-dimensional or lumped-parameter) models, representing each tract between two contact points as an independent mechanical component. In an advantageous embodiment, each tract is represented by a massless spring, and the contact points are represented as masses. Such a mass-spring model is governed by a fast-to-solve second-order partial differential system of equations accounting for mesh deformation under the effect of internal and external forces. Other models may be used.

While the stent deformation is computed using a mass-spring model, vessel deformation due to the stent may computed using finite element modeling. A complete explicit representation of the mesh and the finite element analysis for vessel deformation may be computed efficiently using optimize numerical algorithms and multi-core architectures. In such an approach, the Newton's second law is solved, where the tissue properties are modeled according a constitutive law (e.g., tissue model) and used to compute the stress tensor. External forces, coming from blood pressure or from the contacts with the stent, are added. Stent-wall contacts are automatically detected using a ray-casting or other approach, and a contact force with friction is added at the nodes of contact on both stent and vessel, according to the action-reaction principle. During the deployment, the stent deforms the vessel until reaching an equilibrium, which is automatically detected by monitoring vertex-wise velocities, and the simulation is then stopped.

The physics-based simulation of interaction between the models provides information indicating the effects of the particular therapy model with the personalized model of the patient. The effects are measured as any parameter or parameters, such as structural (e.g., volume, diameter) or hemodynamic (e.g., flow). In the stent example, post-deployment hemodynamics are calculated. The simulation deforms the anatomical model of the vessel with respect to its original shape due to the forces generated by the deployment procedure. The outcome of the selected procedure is predicted by comparing properties inside the vessel before and after the virtual stenting.

The blood vessel wall may be modeled as a rigid or deformable structure. In the latter case, the mechanical properties of the vessel wall after stent deployment may be estimated. A possibility is using the same properties estimated at baseline. If histology information is available at baseline (e.g. calcium score from CT images), the mechanical properties of the wall after deployment may be predicted based on the concentration of calcified tissue and the estimated degree of fragmentation of the plaque. The process of plaque fragmentation may also be simulated as part of the stent deployment simulation. Using a biomechanics model, the load on the stent after deployment may be computed.

The biomechanics model may be coupled to a fluid mechanics model to provide insight on the dynamics response of the vessel after stent implantation, in terms of stability of the stent (potential risk of stent fracture), and other risk factors. For instance, the increased stiffness of the stented tract may cause reflection of pressure waves, and consequently increase the afterload to the right ventricle, with potential long-term adverse effects on the heart. Other types of models may be employed, like lumped one-dimensional solver or rigid-body three-dimensional computational fluid dynamics (CFD) model. The load on the stent may be computed for each time step of the simulation to identify areas that could potentially fracture (higher stress) and other risks.

Acts 46 and 48 represent different approaches for simulation. The different simulation approaches use patient-specific physiology information. Both may use physiological models. Alternatively, the machine-learnt approach for finding similar patients does not use the physiological model. Only one or both approaches may be used. Other approaches may be provided.

In act 46, a guideline is encoded. The guideline is of a particular therapy or of many different therapies that are to be simulated as virtual experiments. The guidelines may be extracted as therapies previously used on one or more other patients, provided by a group or practice (e.g., hospital), or provided by a board as a standard.

The guideline is encoded, such as encoding as decision trees or Markov decision processes. A user may instead specify the experiments to be performed in the form of scripts. The experiment design may be retrieved automatically from a database of already carried out experiments or a clinical case report. Finally, because the experiment design may be expressed in terms of a Markov decision process, a reinforcement learning approach may be used in act 47to learn the experiments to carry out through successive interactions with the user.

In act 48, a search for similar patients is used to simulate many different therapies. The application of therapy on the current patient is simulated by locating past patients with the same therapy. Past patient results for the different therapies are provided as the results of the simulation for the current patient.

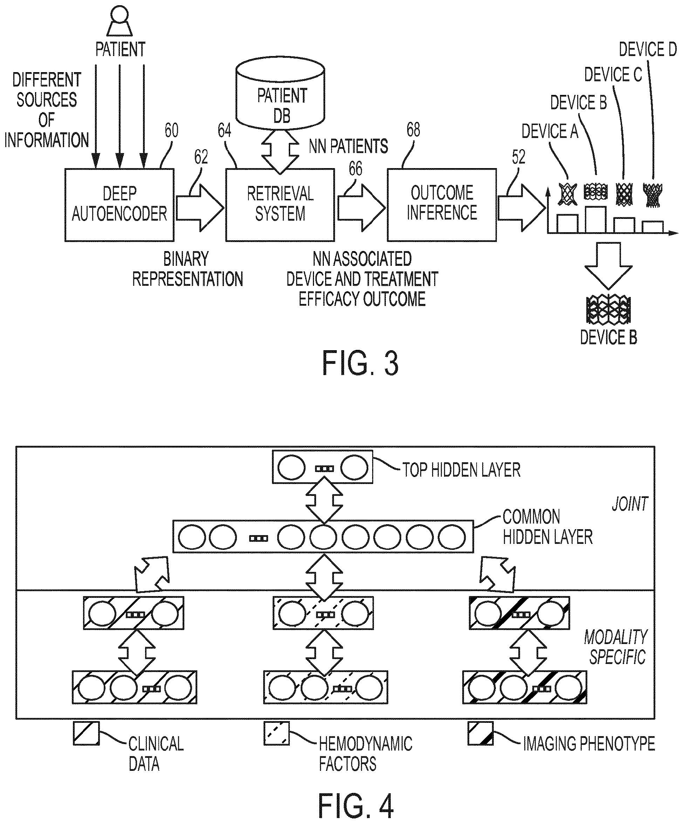

Figure 3 shows an example embodiment of this approach. Other embodiments may be provided, such as using set features to determine nearest neighbor (i.e., similar) past patients. For example, vessel anatomy and/or hemodynamic parameters are used to find past patients with the same or similar characteristics.

A processor performs the acts. The processor accesses a database of past patients. The database of past patients includes patient information for those patients and/or includes representations of the past patient information created with the auto-encoder or other encoding of act 60.

In act 60, patient information is input to a deep auto-encoder. The patient information is from different sources. The different sources provide different types of information, but may be stored in a same memory or device. Alternatively, the different sources are different devices, such as a medical scanner providing image or scan data, a computerized patient medical record database or user interface providing clinical data, and/or the processor itself calculating hemodynamic factors from personalizing a physiological model to segmented data from a scan and/or other measurements.

Any patient information may be used. Figures 3 and 4 show three types of information- clinical data, hemodynamic factors, and imaging phenotype data. Additional, different, or fewer types of information may be input. The patient information is specific to the current patient and the type of therapy or therapy device. For example, information relevant to vessel therapy (e.g., stenting and/or balloon catheterization) for a current patient is extracted and input. The vessel to be stented is segmented or segmented information in input. The flow characteristics through the vessel are input. Values for modeled parameters are input. Diagnosis relevant measures, such as blood pressure or vessel specific pressure, are input. Other data may be input.

The input is used by the deep auto-encoder to determine a representation of the current patient. Usually, learning-based similarity search algorithms consists of: (i) a learned compact representation for patients, (ii) an associated similarity measure that may be eventually learned to be able to compare representations, and (iii) a database of reference patients associated with relevant information (e.g., type of device, size, position, treatment efficacy, and device failure). Patient data may be characterized by multiple sources of information such as clinical and family history, hemodynamic factors before and/or after stent implantation, imaging phenotypes computed from MR, CT and/or ultrasound. For virtual experiments, models, model parameters, and model output are also stored. In this context, defining a suitable patient representation with its associated similarity measure is a challenging problem. Indeed, these different sources of information live in different features spaces that present different dimensionalities, scales, and structures, making use of learning-based similarity search algorithms difficult.

Deep auto-encoding or other approach for reducing the amount of data to represent a specific patient is used. The decision support or case reasoning system builds onto a deep auto-encoder for learning a multimodal low dimensional representation of patient data. The deep auto-encoder is a stack of restricted Boltzmann machines, so may be efficiently pre-trained layer-wise in an unsupervised fashion, and fine-tuned using back-propagation using supervision.

Deep generative models have been used to address the problem of learning a low-dimensional representation of documents. The deep auto-encoder, as trained, creates a digital representation of the input data, providing efficient representation for fast retrieval. Intermediate hidden layers are used to perform dimensionality reduction and mixing the different sources of information. Figure 4 shows a representation with four layers. The first two layers reduce clinical, hemodynamic factors, and imaging phenotype information separately. The top two layers combine this multi-modal information into a binary or digital representation. Other representations, such as using other coding than 1 and 0 may be used.

The deep auto-encoder is pre-trained without using any supervision by using a large set of past patients. Afterwards, either unsupervised or supervised back-propagation is performed to fine-tune the network. In the supervised case, outcome information, such as treatment efficacy, may be used to drive back-propagation.

Once trained, the deep auto-encoder is applied in act 62 to the input patient information for the current patient. The result is a digital data of a lower dimensional representation of the patient information. The patient information is reduced to less information, providing a lower dimensional representation to be used for comparison.

In act 64, the lower-dimensional, digital representation of the current patient is compared to patient information for past patients in a database. The comparison may be with low-dimensional digital representations of the past patients created with the same deep auto-encoder. The digital representations for past patients are created as needed or previously created and stored.

The past patients may be restricted to patients having received the same types of therapy and/or having the same condition or diagnosis. For example, the past patients have been treated with the type or types of therapy devices for which the decision support system is virtually experimenting. The past patients are designated by type of intervention. The interventions are logged using multiple and heterogeneous information like device, device parameters, imaging, or other information. Alternatively, the database includes past patients with many different diagnoses and the comparison is relied on to find the similar patients that are relevant for the type of therapy.

In act 66, the processor selects similar patients to the current patient with an output of the deep auto-encoder. By creating the low-dimensional representation, the auto-encoder finds features of the current patient for comparison to data of the past patients. The clinical, hemodynamic, and medical scan information as reduced to the digital representation allows for matching with past patients. Once encoded using the digital representation, a new incoming patient may be compared to all reference patients within the reference database by Hamming distance or a learned similarity measure. The similarity measure may be learned using the same training set as the auto-encoder. Other similarity measures may be used, such as a hash function for finding approximate matches. The hash function is used to retrieve patients with similar coding.

Any number of patients with sufficient similarity may be identified. The number may be restricted, such as selecting the N most similar past patients. Alternatively, the similarity is thresholded, so only patients with sufficient similarity are selected. A combination of similarity threshold and number of past patients may be used.

In act 68, the output of the simulation experiments is provided from the past patient information. Based on the retrieved nearest neighbor patients, the processor infers outcomes by aggregating the frequency and treatment efficacy for each type of device among the retrieved patients. The best device is the one that maximizes a score derived from that information. A range of outcomes from a range of therapy devices is inferred. The similar patients have been subjected to different therapies, so the corresponding results are used. Any measure of outcome may be used, such as a hemodynamic measure, stent failure, re-admittance, re-treatment, or life expectancy.

The inference indicates the therapy (e.g., stent properties) used for similar patients. The success of the therapy on the current patient is inferred from success or not of the same therapy on past patients. Since the past patients have been subjected to different therapies, the range of therapies and associated distribution of outcomes for each of the therapies is output as the results of the simulation.

Further processing may be performed for the simulation using similar past patients. Figure 5 shows an embodiment combining physics-based simulation with the simulation using similarity to past patients.

In act 70, one or more of the therapy devices are selected based on the simulated outcomes. In the approach of Figure 3, hemodynamic factors are derived for before and after implantation simulation or just before. For the approach of Figure 5, the hemodynamic factors before and after physics-based simulation are separated to avoid the processing for physics-based simulation for all of the possible therapy devices. For acts 60 and 64, the patient representation in the database is based on four or other number of sources of information. For example, clinical data, measured hemodynamic factors before implantation simulation, and imaging phenotype information is input to the auto-encoder.

The hemodynamic factors resulting from physics-based simulation are used for a subsequent similarity identification. The previous digital representation is augmented with a digital code generated by an auto-encoder using the hemodynamic factors after implantation simulation. In a first pass, nearest patients are retrieved in act 66 from the database using the digital representation. In act 70, the therapies (e.g., therapy devices) with the desired outcomes are then selected and used for performing implantation simulation in act 72. Treatment with the therapy devices is simulated with the physiological model fit to the current patient. The same or different patient information input to the encoder in act 60 is used to fit the physiological model. The processor computationally solves for the interaction of the therapy model with the personalized physiological model. The therapy model is specific to the therapy or therapy device selected in act 70.

In act 74, the hemodynamic factors resulting from simulation of the therapy are calculated. The derived simulated hemodynamic factors are used to build an augmented digital representation in act 76. The patient information used in act 60 and the calculated post-treatment hemodynamic factors from the simulation are input to the separately trained auto-encoder. The same or different patient information may be input. Alternatively, the calculated hemodynamic factors are input without the other information and the results are added to the previous digital representation. Other factors than hemodynamic factors may be computed from the model or in the therapy simulation.

Afterwards, the nearest past patients and experiment designs are retrieved in act 78 using this augmented representation. Another set of similar patients is selected based on the digital representation output by the autoencoder. The selection criteria (e.g., similarity threshold) is the same or different than for act 66. The resulting set of past patients is the same or different.

The outcomes are inferred from the selected past patients in act 80. The inference provides a range of outcomes for each of the therapies selected in act 70. Alternatively, a median, mean, or other statistical outcome information is inferred. The same or different inference is used in act 80 as for act 68. For example, the inference in act 68 is directed to mean outcome while the inference in act 80 is directed to the distribution of outcomes. Different parameters may be used, such as measuring outcome differently for the two acts 68 and 80.

Reinforcement learning of act 47 (Figure 2) may be used to improve the experiment design. The decision support system learns the optimal or a variety of treatment options from the user. Machine learning techniques are used to automatically identify the best or other options among the available alternatives by evaluating and comparing the output of the different therapy simulations. This, however, assumes that there exist clear, objective criteria for the "goodness" of a simulation. If such criteria do not exist, a database of expert decisions (e.g. from previous choices of clinicians on similar patients) is used to automatically learn such criteria "from experience." To this end, inverse reinforcement learning is applied.

The simulations to be performed are defined, in part, by a "policy" (guideline) computed using reinforcement learning in act 47. Because the design of successive experiments may be setup according to a set of rules, where each virtual experiment may be seen as an "action", and because the outcomes of previous experiments may be seen as "states" of the decision process, the design of experiments may be encoded as a Markov decision process. An optimal policy may be computed from the Markov decision process using, for example, dynamic programming or more advanced reinforcement learning techniques such as Q-Learning. During the learning procedure, an agent repeatedly tries different actions from the set of available actions to gain experience, which is used to learn an optimal policy. A policy determines for any possible state during the decision process the best action to perform in order to maximize future rewards. The rewards are set up in a way such that positive rewards are given for actions that lead to fast and reliable (low uncertainty) decision making with few virtual experiments to carry out, while negative rewards are given for experiments which provide little or no value to the decision making process. Experience from past decision making processes may be used to define the rewards and the states, and the experiments performed on past patients may be used to define the set of actions.

In Figure 1, the reinforcement learning is part of act 44. Which experiments to carry out is determined by the reinforcement learning. In the example of Figure 5, acts 70-74 may use reinforcement learning to determine different implantation simulation experiments to carry out based on information from similar patients.

Referring again to Figure 1, the processor estimates uncertainties in the simulation of the different therapies in act 50. The uncertainty is in the outcome, in the range of outcomes, in the input information used in simulation, in the models, in the simulation, in other sources, or combinations thereof. Uncertainty may not be calculated for some of the experiments, such as not calculating uncertainty for a sub-set of the simulated therapies. In alternative embodiments, uncertainty is not calculated.

Any estimation of uncertainty may be used. For example, one or more of the models used in a simulation may have a pre-determined uncertainty, such as based on testing of the model. The model-based uncertainties are used as the estimates for the uncertainty of the results (e.g., uncertainty of the efficacy of a simulated therapy). In another example, variability of the results is used as a measure of the uncertainty. Greater variability in the outcome for a given simulation indicates greater uncertainty. In the stent example using similar patients to simulate, similar patients having a broad range or greater distribution throughout the range indicates more uncertainty than the distribution being narrow and/or more strongly in a narrow range.

In one embodiment, the decision support system estimates predicted parameters uncertainty due to noise in the data and also model assumptions. The uncertainty in one or more parameters used as inputs or provided as outputs is determined. For example, the option to display a confidence indicator for each presented data, which may be of particular importance for the therapy decision, is provided. This indicator may be a confidence interval or a numerical value (e.g. between 0 and 1 for low or high confidence) or a colored button (e.g. red, yellow and green for low, medium or high confidence). The uncertainty information itself may either be provided directly by the models or indirectly computed. For indirect computation, the application program interface of the therapy model is accessed to trigger simulation. Using uncertainty quantification methods, the uncertainty in the predicted therapy outcome is estimated, for instance, by varying the model input parameters in a specified range and computing the variability in the outcome among the different simulations. Results that fall within the same uncertainty area may be gathered automatically. Results may then be ranked according to predicted therapy outcome but also the confidence in the calculated parameters.

In act 52, the results are presented to the user. The therapy options and simulated results are output by the processor on a display. The output information may have any format, such as a list, chart, graph, or icons. Each of the virtual experiment results are output, such as for each of the therapies. Alternatively, a sub-set of the therapies (e.g., better outcomes) and corresponding statistics are output. The outcomes may be clustered or ranked, and the output presented with the clustering or ranking.

The results, such as the range, median, mean, and/or other result statistic, are provided for each of the therapy devices, types of therapy, models used, or combinations thereof. Different measures of outcome may be presented for each therapy. Additional, different, or less information may be provided.

The results may be presented with estimated uncertainties, if available. For example, an uncertainty rank or percentage value is output with the results. The results may be color coded to indicate degree of uncertainty.

Different sets of results may be presented. For example, results from similar patients are output based on act 68 of Figure 5. Another set of results are output based on a different set of similar patients in act 80 are output as well. Alternatively, final or last occurring results are presented.

The output may include the range of outcomes for each therapy or therapy device for each of a plurality or range of therapies or therapy devices. Statistics about therapy outcome based on the range of therapies may be output, such as showing a trend in improving outcomes with variation in a given parameter in the therapy (e.g., trend in efficacy with increasing diameter of stent).

In alternative embodiments, the decision support system learns to classify the model output from the model input. The time and processor-consuming simulation process may be avoided by training a classifier to predict the output given the input information. The training uses any machine learning from a large database of patient data or, if not available, by generating synthetic data through simulation on randomly generated geometries and physiological conditions. In other embodiments, the decision support system learns through reinforcement the best therapy options directly, without the need of performing the virtual experiment. Alternatively, the decision support system learns the models and the errors from actual observations. When queried, the system first outputs the results from its learned features, then based on the expected error, queries one or more an additional simulations to refine the results.

Figure 6 shows one embodiment of a system for decision support in therapy. The system is a computer, controller, workstation, server, or other arrangement. The system includes the display 14, memory 16, and processor 18. Additional, different, or fewer components may be provided. In other embodiments, the medical imaging system 11 is part of the system. In yet other embodiments, a picture archiving and communications system (PACS) or other memory is provided instead of or in addition to the medical imaging system 11 for supplying images.

In one embodiment, the processor 18 and memory 16 are part of a server hosting the therapy decision support system for use by a computer as the client. The client and server are interconnected by a network, such as an intranet or the Internet. The client may be a computer of the medical imaging system 11 or a computer of a medical professional, and the server may be provided by a manufacturer, provider, host, or creator of the therapy decision support system.

The medical imaging system 11 is any now known or later developed imaging system. For example, the medical imaging system 11 is a computed tomography, ultrasound, x-ray, magnetic resonance, or functional imaging system. As a computed tomography system, an x-ray source and detector are mounted on or in a gantry on opposite sides of a patient space and corresponding patient bed. As the gantry moves the source and detector around the patient, a sequence of x-ray projections of the patient are acquired. A processor, such as the processor 18 or a different processor, reconstructs the x-ray attenuation in three-dimensions or for one or more slices. As an ultrasound system, a transducer and beamformers are used to scan the patient. A processor, such as the processor 18 or a different processor, reconstructs the echoes in three-dimensions or for one or more slices.

The display 14 is a CRT, LCD, projector, plasma, printer, smart phone or other now known or later developed display device for displaying the images, illustrations of a personalized model, illustrations of a therapy device or therapy, hemodynamic or other parameters, indication of similarity, simulation video, results of simulation, outcome, uncertainties, and/or other information. For example, the display 14 displays a list of therapies for similar patients or used in virtual experiments, outcome for each of the therapies (e.g., median, mean, or ranges of outcomes for each of the therapies), and/or uncertainty. A therapy or therapies (e.g., range of stent sizes, shapes, and/or materials and/or placement) recommended for a given patient may be indicated to the physician. Based on user selection, a video of the simulation of the selected therapy is shown.

The patient information, segmented information, models, personalized models, past patient information, lower-dimensional representation, nearest neighbor selections, simulation results, hemodynamic factors, and/or other information are stored in a non-transitory computer readable memory, such as the memory 16. The memory 16 is an external storage device, RAM, ROM, database, and/or a local memory (e.g., solid state drive or hard drive). The same or different non-transitory computer readable media may be used for instructions and other data. The memory 16 may be implemented using a database management system (DBMS) managed by the processor 18 and residing on a memory, such as a hard disk, RAM, or removable media. Alternatively, the memory 16 is internal to the processor 18 (e.g. cache).

The instructions for implementing the decision support system, simulation, or other processes, methods and/or techniques discussed herein are provided on non-transitory computer-readable storage media or memories, such as a cache, buffer, RAM, removable media, hard drive or other computer readable storage media (e.g., the memory 16). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU or system. Because some of the constituent system components and method steps depicted in the accompanying figures may be implemented in software, the actual connections between the system components (or the process steps) may differ depending upon the manner in which the present embodiments are programmed.

A program may be uploaded to, and executed by, the processor 18 comprising any suitable architecture. Likewise, processing strategies may include multiprocessing, multitasking, parallel processing and the like. For simulation, a graphics-processing unit and/or multi-core processor may be used. The simulation, such as finite element analysis, includes repetitive calculations making parallel processing architectures more efficient. The processor 18 is implemented on a computer platform having hardware, such as one or more central processing units (CPU), a random access memory (RAM), and input/output (I/O) interface(s). The computer platform also includes an operating system and microinstruction code. The various processes and functions described herein may be either part of the microinstruction code or part of the program (or combination thereof) which is executed via the operating system. Alternatively, the processor 18 is one or more processors in a network.

## Claims

1. A computer-implemented method for decision support for therapy, the method comprising:
inputting patient information from different sources to a first deep auto-encoder, the patient information specific to a first patient and a type of therapy device;
selecting similar patients to the first patient with an output of the first deep auto-encoder, the similar patients having been treated with the type of therapy device;
inferring a range of outcomes from a range of therapy devices of the type of therapy device from data for the similar patients,
wherein set features are used to determine nearest neighbor past patients, and wherein based on the retrieved nearest neighbor patients, the processor infers outcomes by aggregating the frequency and treatment efficacy for each type of device among the retrieved patients; and
displaying the range of outcomes and the range of therapy devices for the first patient,
wherein the decision support is for a use case of pulmonary artery stenting and/or cardiac resynchronization planning and/or ablation therapy and/or valve replacement,
wherein the patient information includes scan data of a medical scanner, wherein the scan data represents a two or three-dimensional region of the patient, wherein the scan is repeatable to represent the region over time.

2. The method of claim 1 further comprising estimating uncertainties of the outcomes, wherein displaying comprises displaying the outcomes with the uncertainties.

3. The method of claim 1 or 2 wherein inputting comprises inputting the patient information as clinical data, hemodynamic factors, and imaging data.

4. The method according to any of claims 1 to 3 wherein selecting comprises selecting with the output being a digital representation of a lower dimensional representation of the patient information.

5. The method according to any of claims 1 to 4 further comprising simulating treatment of the therapy devices with a physiological model fit with at least some of the patient information, inputting the patient information and results of the simulation of the treatment to a second deep auto-encoder, and selecting another set of similar patients based on an output of the second deep auto-encoder.

6. The method according to any of claims 1 to 5 wherein the type of therapy device comprises a stent, the patient information includes vessel information from a medical scanner, and the range of therapy devices comprises stents with different properties.

## Patentansprüche

1. Ein computerimplementiertes Verfahren zur Entscheidungsunterstützung für eine Therapie, wobei das Verfahren umfasst:
Eingabe von Patienteninformationen aus verschiedenen Quellen in einen ersten tiefen Auto-Kodierer, wobei die Patienteninformationen für einen ersten Patienten und einen Typ von Therapiegerät spezifisch sind ;
Auswahl von Patienten, die dem ersten Patienten ähnlich sind, mit einer Ausgabe des ersten tiefen Auto-Kodierers, wobei die ähnlichen Patienten mit der Typ von Therapiegerät behandelt worden sind ;
Ableitung einer Reihe von Ergebnissen aus einer Reihe von Therapiegeräten des Typs von Therapiegerät aus den Daten für ähnliche Patienten,
wobei die eingestellten Merkmale verwendet werden, um die nächstgelegenen Patienten aus der Vergangenheit zu bestimmen, und wobei der Prozessor auf der Grundlage der abgerufenen nächstgelegenen Patienten Ergebnisse ableitet, indem er die Häufigkeit und die Behandlungswirksamkeit für jeden Typ von Therapiegerät unter den ausgewählten Patienten zusammenfasst; und
Anzeigen der Reihe von Ergebnissen und der Reihe von Therapiegeräten für den ersten Patienten,
wobei die Entscheidungsunterstützung für einen Anwendungsfall von Pulmonalarterien-Stenting und/oder kardialer Resynchronisationsplanung und/oder Ablationstherapie und/oder Klappenersatz ist,
wobei die Patienteninformationen Scandaten eines medizinischen Scanners aufweisen, wobei die Scandaten eine zwei- oder dreidimensionale Region des Patienten darstellen, wobei der Scan wiederholbar ist, um die Region über die Zeit darzustellen.

2. Verfahren nach Anspruch 1 ferner umfassend das Schätzen von Unsicherheiten der Ergebnisse, wobei das Anzeigen die Darstellung der Ergebnisse mit den Unsicherheiten umfasst.

3. Verfahren nach Anspruch 1 oder 2, wobei das Eingeben das Eingeben der Patienteninformationen als klinische Daten, hämodynamische Faktoren und Bildgebungsdaten umfasst.

4. Verfahren nach einem der Ansprüche 1 bis 3, wobei das Auswählen ein Auswählen umfasst, welches die Ausgabe einer digitalen Darstellung einer niederdimensionalen Darstellung der Patienteninformation aufweist.

5. Verfahren nach einem der Ansprüche 1 bis 4 ferner umfassend das Simulieren der Behandlung der Therapiegeräte mit einem physiologischen Modell, das an mindestens einige der Patienteninformationen angepasst ist, das Eingeben der Patienteninformationen und der Ergebnisse der Simulation der Behandlung in einen zweiten tiefen Auto-Kodierer und das Auswählen eines weiteren Satzes ähnlicher Patienten auf der Grundlage einer Ausgabe des zweiten tiefen Auto-Kodierers.

6. Verfahren nach einem der Ansprüche 1 bis 5, wobei der Typ des Therapiegeräts einen Stent umfasst, die Patienteninformation Gefäßinformationen von einem medizinischen Scanner aufweist und die Auswahl an Therapiegeräten Stents mit unterschiedlichen Eigenschaften umfasst.

## Revendications

1. Procédé mis en oeuvre par ordinateur pour une aide à la décision pour une thérapie, le procédé comprenant :
l'entrée d'informations sur le patient provenant de différentes sources dans un premier codeur automatique profond, les informations sur le patient étant spécifiques à un premier patient et à un type de dispositif de thérapie ;
sélectionner des patients similaires au premier patient avec une sortie du premier codeur automatique profond, les patients similaires ayant été traités avec le type de dispositif de thérapie ;
déduire une gamme de résultats d'une gamme de dispositifs de thérapie du type de dispositif de thérapie à partir de données pour les patients similaires,
dans lequel les caractéristiques de l'ensemble sont utilisées pour déterminer les anciens patients voisins les plus proches, et dans lequel, sur la base des patients voisins les plus proches récupérés, le processeur déduit des résultats en agrégeant la fréquence et l'efficacité du traitement pour chaque type de dispositif de thérapie parmi les patients récupérés ; et
afficher la gamme de résultats et la gamme de dispositifs de thérapie pour le premier patient,
dans lequel l'aide à la décision est destinée à un cas d'utilisation de stenting d'artère pulmonaire et/ou de planification de resynchronisation cardiaque et/ou de thérapie d'ablation et/ou de remplacement de valve,
dans lequel les informations sur le patient comprennent des données de balayage d'un scanner médical, dans lequel les données de balayage représentent une région bi- ou tridimensionnelle du patient, dans lequel le balayage peut être répété pour représenter la région dans le temps.

2. Procédé selon la revendication 1 comprenant en outre l'estimation des incertitudes des résultats, dans lequel l'affichage comprend l'affichage des résultats avec les incertitudes.

3. Procédé selon la revendication 1 ou 2, dans lequel l'entrée comprend l'entrée des informations du patient sous forme de données cliniques, de facteurs hémodynamiques et de données d'imagerie.

4. Procédé selon l'une quelconque des revendications 1 à 3, dans lequel la sélection comprend la sélection avec la sortie étant une représentation numérique d'une représentation de dimension inférieure des informations du patient.

5. Procédé selon l'une quelconque des revendications 1 à 4, comprenant en outre la simulation du traitement des dispositifs de thérapie avec un modèle physiologique adapté à au moins certaines des informations sur le patient, l'entrée des informations sur le patient et des résultats de la simulation du traitement dans un deuxième codeur automatique profond, et la sélection d'un autre ensemble de patients similaires sur la base d'une sortie du deuxième codeur automatique profond.

6. Procédé selon l'une quelconque des revendications 1 à 5, dans lequel le type de dispositif de thérapie comprend un stent, les informations sur le patient comprennent des informations sur les vaisseaux provenant d'un scanner médical, et la gamme de dispositifs thérapeutiques comprend des stents ayant des propriétés différentes.
